# EUROPEAN PATENT SPECIFICATION

(11) **EP 2 240 597 B1**
(45) Date of publication and mention of the grant of the patent: **07.08.2013**
(21) Application number: 09702254.5
(22) Date of filing: 21.01.2009
(51) Int. Cl.: A61N 5/06, C12Q 1/04, G01N 21/91

(54) **SELECTIVE PHOTOSTIMULATION TO INDUCE CELL PROLIFERATION**
SELEKTIVE PHOTOSTIMULATION ZUR HERBEIFÜHRUNG EINER ZELLPROLIFERATION
PHOTOSTIMULATION SÉLECTIVE DESTINÉE À INDUIRE UNE PROLIFÉRATION CELLULAIRE

(30) Priority: 18.01.2008 US 22202 P
(43) Date of publication of application: 20.10.2010
(73) Proprietor: The General Hospital Corporation, Boston, MA 02114 (US)
(72) Inventor: LATINA, Mark A., North Andover Massachusetts 01845 (US); YARMUSH, Martin L., Newton Massachusetts 02459 (US)
(74) Representative: Peterreins, Frank
(86) International application number: PCT/US2009/031599
(87) International publication number: WO 2009/092112

(56) References cited:
- US-A- 5 549 596
- US-A- 5 549 596
- US-A- 6 059 772
- US-A1- 2001 034 319
- US-A1- 2003 050 678
- US-A1- 2006 074 468
- US-A1- 2006 161 145
- US-A1- 2007 213 693
- US-B1- 7 036 516

## Description

### TECHNICAL FIELD

This invention relates to photo-treatment of tissue, and more particularly to the use of photostimulation to induce cell proliferation.

### BACKGROUND

Treatment of tissue with light of, for example, a laser, is common practice in various medical fields. In ophthalmology, for example, applications include the use of a laser for photocoagulation, i.e., the irradiation of light causing tissue damage. Photocoagulation is used to treat retinal disorder diseases such as age-related macular degeneration (AMD), diabetic maculopathy (DMP), proliferative diabetic retinopathy (PDR), a central serous retinopathy (CSR). In addition, U.S. Patent No. 5,549,596 describes the use of selective laser targeting as a method of damaging pigmented intraocular cells while sparing adjacent nonpigmented cells.

### SUMMARY

The invention relates to a system as described in claim 1.

The invention is based, at least in part, on the discovery that if a tissue is irradiated with a sublethal dose of radiation, e.g., from a laser, that pigmented cells in the tissue are selectively stimulated (i.e., photostimulated) to proliferate and to produce higher levels of certain mitogenic factors and growth factors such as platelet-derived growth factor (PDGF). Thus, the sublethal radiation, when properly applied, can cause a cellular biochemical response, which induces proliferation and growth, and thereby results in a healing effect on atrophied or diseased tissue, while avoiding cell damage and cell death.

In particular, the various parameters of a pulsed radiation, such as power, pulse duration (which is also referred to herein as "pulse width"), total radiation energy (which is also referred to herein as "fluence"), wavelength, and if multiple pulses are used, the pulse rate and total number of pulses, are carefully selected and controlled to minimize killing the irradiated pigmented cells. For example, the duration of the sublethal laser pulse, or pulses, should be less than the thermal relaxation time of individual cells in the tissue, and the wavelength used should be close to the absorbance maximum of the cells to be targeted, or of a pigment within those cells.

As an example, methods are described that involve selectively photostimulating pigmented cells, such as intraocular cells, e.g., trabecular meshwork (TM) cells, retinal pigmented epithelial cells, uveal pigmented cells, melanoma cells, or conjunctival pigmented cells. The pigmented cells may contain endogenously synthesized pigment, or may be cells, e.g., phagocytic cells, into which exogenous pigment is introduced by contacting the phagocytic cells with exogenous pigment before irradiating the area of tissue containing the cells. Alternatively, pigments can be introduced to non-phagocytic cells by genetic engineering techniques or by passing the pigments through the cell membrane.

As used herein, endogenous pigment refers to pigment synthesized and retained within a cell, and exogenous pigment refers to pigment within a cell that was not synthesized within the same cell.

In some embodiments, the phagocytic cell is a trabecular meshwork cell and exogenous pigment is introduced into the aqueous humor by laser irradiation of the iris.

The new methods involve irradiating tissue with a "sublethal" fluence or total dose of radiation administered in one treatment session that involves the administration of one or a short series of pulses administered so as to photostimulate the pigmented cells in the tissue, while keeping a significant percentage (over 90%) of the total cells within the irradiated tissue alive, as measured by a live/dead viability/cytotoxicity assay.

The sublethal fluence varies for examples that are not part of the invention for different powers, wavelengths, pulse durations, and repetition rates, as well as for different laser and cell types and levels of pigmentation in the cells. For example, for a single pulse irradiation of RPE cells with pulse durations between 250 ns and 3 µs, the sublethal fluence can be in the range of from about 5 to 8 mJ/cm² to about 250 to 285 mJ/cm², e.g., about 10 to 220, 25 to 200, and 55 to 110 mJ/cm². For example, a fluence of less than 120 mJ/cm² for a treatment session of one 1 µs pulse at 590 nm is a sublethal fluence using a pulsed dye laser on RPE cells. As a further example, for a single pulse irradiation of RPE cells with pulse durations below 250 ns, e.g., pulse durations of 10-20 ns, the sublethal fluence can be in the range of from about 0.1 to 1 mJ/cm² to about 20 to 30 mJ/cm², e.g., about 0.5 to 20, 1 to 15, and 5 to 10 mJ/cm².

As used herein, selective photostimulation of cells is an effect induced by a laser that is operated at a wavelength preferentially absorbed by a specific pigment in pigmented cells compared to unpigmented (also referred to herein as nonpigmented) cells. The effect is to cause the irradiated pigmented cells to have a biochemical response that increases production of certain mitogenic and/or growth factors such as, for example, one or more of Platelet-Derived Growth Factor (PDGF), Transforming Growth Factor Beta (TGFβ), Basic Fibroblast Growth Factor (bFGF), Epidermal Growth Factor (EGF), Insulin-like Growth Factor (IGF), Vascular Endothelial Growth Factor (VEGF), Pigment Epithelium-Derived Factor (PEDF), and heat stock proteins, and/or to cause the irradiated cells to increase their level of proliferation compared to non-irradiated and unpigmented cells.

As used herein, an unpigmented or nonpigmented cell is a cell that has a level of a specific pigment that is less than half of the level found in a given pigmented cell.

As an example are described methods of selectively photostimulating pigmented cells in a tissue in a patient that include selecting a region of tissue containing a pigmented target cell and a nonpigmented cell, wherein the pigment is either endogenously synthesized or exogenous pigment, and irradiating the tissue with one or more radiation pulses, e.g., from a laser, wherein each pulse comprises (i) a wavelength that is absorbed more in the pigmented cell than in the nonpigmented cell, and (ii) a pulse duration that is shorter than a thermal relaxation time of the pigmented cell, and wherein the total radiation energy applied provides a sublethal fluence to the pigmented target cells, thereby selectively photostimulating pigmented cells in the tissue.

Examples of the methods can include one or more of the following features and/or features of other aspects described herein. For example, in some embodiments, the sublethal fluence of the total laser radiation can be below 120 mJ/cm² and the pulse duration is in the range from 0.5 µs to 8 µs. In some embodiments, the radiation can have a wavelength in the visible spectrum (e.g., in a range from about 400 nm to about 800 nm).

In certain embodiments that are not part of the invention, the laser radiation can be delivered in one or more pulses, each with a pulse duration of between about 1 ns and about 2 µs. In some embodiments, the laser fluence of the total laser radiation can be below 120 mJ/cm².

In some embodiments that are not part of the invention, the sublethal laser fluence of the total laser radiation can be below 20 mJ/cm² and the pulse duration can be about 10 nanoseconds. The radiation can have a wavelength in the visible spectrum (e.g., in a range from about 400 nm to about 800 nm).

In other embodiments that are not part of the invention, the sublethal laser fluence of the total laser radiation can be below 200 mJ/cm² and the pulse duration can be about 10 nanoseconds and the radiation can have a wavelength in the infrared spectrum (e.g., in the range from 1000 nm to 1500 nm).

The methods that are not part of the invention can include a subsequent irradiation step or steps after a regeneration period.

In some embodiments, the one or more laser radiation pulses can impinge upon the tissue in a target spot having a diameter of between about 0.05 and about 1.5 mm or about 0.1 and about 1.0 mm.

In various embodiments, the tissue can include a melanoma or intraocular tissue, e.g., a trabecular meshwork cell, a retinal pigmented epithelial cell, a uveal pigmented cell, and/or a melanoma cell. In some embodiments, the pigmented cell can be a phagocytic cell within an intraocular area into which the pigment is introduced by contacting the phagocytic cell with an exogenous pigment before irradiating the area.

As an example are described methods of selectively inducing proliferation of retinal pigment epithelial cells that include selecting a region of a retina containing retinal pigment epithelial cells, wherein the retinal pigment epithelial cells contain pigment which is either endogenously synthesized or is exogenous pigment, and irradiating the region of retinal pigment epithelial cells with one or more sublethal radiation pulses, wherein each pulse provides a fluence of less than 120 mJ/cm² and comprises a pulse duration of at least 0.5 µs at a wavelength between 400 nm and 800 nm or a pulse duration in the range from 5 ns to 0.5 µs at a wavelength between 1000 nm and 1500 nm and a wavelength that is absorbed more in the retinal pigment epithelial cells than in surrounding tissue; wherein individual pulses are applied with a sufficient separation of time to ensure no photocoagulation of the tissue; and wherein the total laser radiation energy applied to the tissue provides a sublethal laser fluence to the retinal pigment epithelial cells, thereby selectively inducing proliferation of the retinal pigment epithelial cells in the tissue.

Embodiments of the methods that are not part of the invention can include one or more of the following features and/or features of other aspects described herein. For example, in some embodiments, the laser radiation can impinge upon the intraocular area in a target spot of between about 0.05 and about 1.5 mm in diameter or between about 0.1 and about 1.0 mm in diameter.

In some embodiments, the radiant exposure can be below about 20 mJ/ cm² at a pulse duration in the range from 5 ns to 0.5 ms at a wavelength between 400 nm and 800 nm. In certain embodiments, the methods further include introducing exogenous pigment into one or more of the retinal pigment epithelial cells.

As an example are described methods of selectively photostimulating pigmented cells in a tissue in a patient that include selecting a region of tissue containing a pigmented target cell and a nonpigmented cell, wherein the pigment is either endogenously synthesized or exogenous pigment, selecting a pulse duration of one or more radiation pulses, which is shorter than a thermal relaxation time of the pigmented cell, and a wavelength for generating one or more radiation pulses that is absorbed more in the pigmented cell than in the nonpigmented cell, selecting a fluence generated with the one or more radiation pulses at the selected wavelength and selected pulse duration that is a sublethal fluence to the pigmented cells, and irradiating the tissue with the one or more radiation pulses, wherein the total radiation energy applied to the tissue provides a sublethal fluence to the pigmented target cells, thereby selectively photostimulating pigmented cells in the tissue. Embodiments of these methods that are not part of the invention can include one or more of the features of other aspects described herein.

As an example are described methods of selectively inducing proliferation of retinal pigment epithelial cells that include selecting a region of a retina containing retinal pigment epithelial cells, wherein the retinal pigment epithelial cells contain pigment which is either endogenously synthesized or is exogenous pigment, selecting a pulse duration of one or more radiation pulses and a wavelength for generating the one or more radiation pulses that is absorbed more in the retinal pigment epithelial cells than in surrounding tissue, selecting a fluence generated with the one or more radiation pulses at the selected wavelength and selected pulse duration is a sublethal fluence to the retinal pigment epithelial cells, and irradiating the region of retinal pigment epithelial cells with the one or more radiation pulses, thereby selectively inducing proliferation of the retinal pigment epithelial cells in the tissue. Embodiments of these methods that are not part of the invention can include one or more of the features of other aspects described herein.

In general, in a further aspect, the invention features systems for selectively photostimulating pigmented cells in a tissue in a patient that include a light source for generating one or more radiation pulses, wherein each pulse comprises (i) a wavelength that is absorbed more in the pigmented cell than in the nonpigmented cell, and (ii) a pulse duration that is shorter than a thermal relaxation time of the pigmented cell, an optical system for directing the one or more pulses to a region of tissue containing a pigmented target cell and a nonpigmented cell, wherein the pigment is either endogenously synthesized or exogenous pigment, and a control unit configured to control the irradiating of the tissue with the one or more radiation pulses such that the total radiation energy applied to the tissue provides a sublethal fluence to the pigmented target cells, thereby selectively photostimulating pigmented cells in the tissue. Embodiments of these systems can include one or more of the following features and/or features of other aspects described herein.

In some embodiments, the control unit can be further configured to control at least one of the pulse duration, the wavelength, and the fluence to ensure the sublethal fluence to the pigmented target cells. In certain embodiments, the systems can further include a monitoring unit to monitor the duration, the wavelength, and the fluence of the emitted pulse. The light source can be configured to generate pulses at a duration in the range of nanoseconds. The light source is configured to generate pulses at a wavelength in the visible spectrum in the range of about 532 nm. The systems are configured to generate a radiation fluence at the tissue in the range from 100 mJ/cm² to 250 mJ/cm².

In general, in a further aspect, the invention features systems for selectively inducing proliferation of retinal pigment epithelial cells that include a light source for generating one or more radiation pulses, means for selecting a region of a retina containing retinal pigment epithelial cells, wherein the retinal pigment epithelial cells contain pigment which is either endogenously synthesized or is exogenous pigment, means for selecting a pulse duration of the one or more radiation pulses and a wavelength for generating the one or more radiation pulses that is absorbed more in the retinal pigment epithelial cells than in surrounding tissue, means for selecting a fluence generated with the one or more radiation pulses at the selected wavelength and selected pulse duration is a sublethal fluence to the retinal pigment epithelial cells, and optics for irradiating the region of retinal pigment epithelial cells with the one or more radiation pulses, thereby selectively inducing proliferation of the retinal pigment epithelial cells in the tissue. Embodiments of these systems can include one or more of the features of other aspects of the inventions described herein.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

In case of conflict, the present specification, including definitions, will control. In addition, the materials, methods, and examples are illustrative only and not intended to be limiting.

Other features and advantages of the invention will be apparent from the following detailed description, and from the claims.

### BRIEF DESCRIPTION OF THE DRAWINGS

FIG. 1 is a diagram of a laser apparatus for photostimulation of tissue.
FIG 2 is a flow diagram illustrating photostimulation.
FIG 3 is a diagram of the viability of pigmented RPE cells as a function of laser fluence and melanin content.
FIGs. 4A to 4H are a series of representations of microphotographs of RPE cells (4A-4G) and a graph (4H) illustrating RPE cell regeneration following laser irradiation.
FIGs. 5A to 5E are a series of representations of microphotographs of cells in an in vitro wound healing model (5A-5D) and a graph (5E) illustrating various levels of wound healing over time.
FIGs. 6A to 6E are a series of images of cells (6A-6D) and a graph (6E) illustrating BrdU staining.
FIG. 7 is an illustration of RT-PCR analysis of PDGFA, PDGFB, PDGF receptor alpha (PDGFR-α), and PDGFR-*β* in sham control and laser irradiated RPE cells.
FIG. 8A is an illustration of RT-PCR showing gene expression of PDGFA and PDGFB and their receptors at different times after laser irradiation.
FIG. 8B is an illustration of RT-PCR showing gene expression of TGF*β*1, bFGF, IGF, EGF, VEGF, PEDF, and their receptors, as well as expression of various heat shock proteins and β-actin at different times after laser irradiation.
FIG. 9A is a graph of normalized viability of human fetal RPE (hfRPE) cells as a function of the fluence of a single pulse generated with a Nd:YAG laser.
FIG. 9B is a graph of normalized viability of hfRPE cells as a function of the fluence of a single pulse generated with a dye laser.

Like reference symbols in the various drawings indicate like elements.

### DETAILED DESCRIPTION

Presently, there are a variety of commercially available laser systems for ophthalmic use, any of which can be adapted to perform the methods described and claimed herein. The commercially available ophthalmic surgical lasers can readily be modified, by anyone skilled in laser technology, to produce the requisite, wavelength, target spot size, and sublethal fluence to provide laser-induced selective photostimulation of pigmented cells.

### Laser Systems for Photostimulation

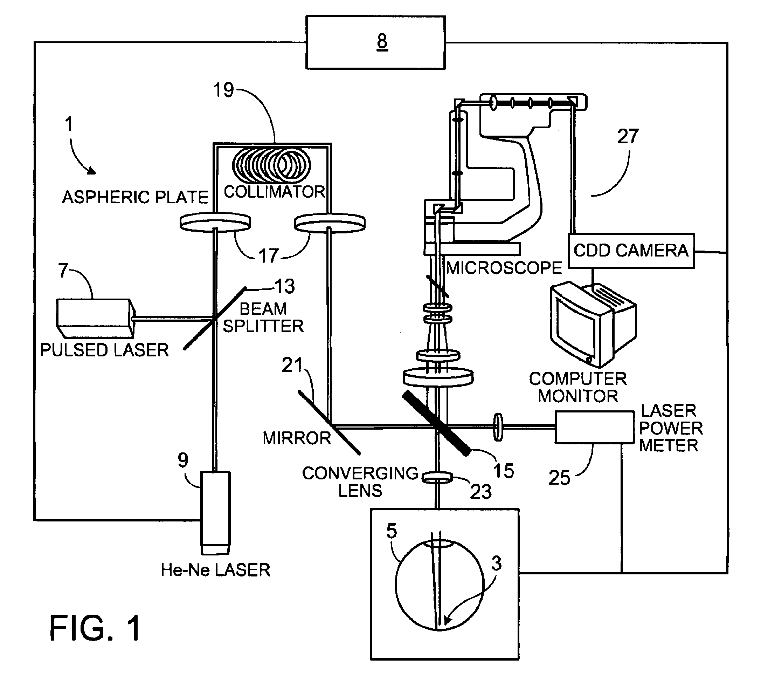

FIG. 1 shows an exemplary system **1** that provides laser pulses for photostimulation of tissue. For example, radiation of the system **1** can be directed to a retina **3** of a human eye **5** to photostimulate the retinal pigment epithelial (RPE) cells, causing an increase in proliferation.

The system **1** includes a pulsed laser source **7** (e.g., a q-switched Nd:YAG or ruby laser), an aiming laser **9,** and optical elements to direct the treatment beam of the laser source 7 and the aiming beam of the aiming laser **9** to a target tissue, e.g. the RPE cells. The optical elements include, for example, beam splitters **13, 15,** an aspheric plate **17,** a beam collimator **19** (optical fiber), mirrors **21,** and lenses, for example, a converging lens **23.**

System 1 further includes a control unit **8** for controlling the laser source 7 (pulse energy, pulse width, number of pulses), the aiming laser **9,** the optical elements (e.g., lens **23),** the position of the target (e.g., retina **3).** Control unit **8** can be configured for automatic and/or manual control. For example, the control unit controls the wavelength of the emitted radiation, the duration of the emitted laser pulses, and the fluence of the emitted radiation in dependence of the activated operation mode. In some embodiments, control unit **8** can also be used to control the position and size of the laser beam at the retina (e.g., manually by the physician). Laser beam parameters can be monitored with, for example, a laser power meter **25.** Moreover, the tissue and the focus spot size can be observed and measured using a microscope **27** coupled to the control unit **8.**

While in FIG. 1 system **1** includes the laser source **7,** in some embodiments, other types of light sources such as an incoherent light source can be used to generate radiation pulses.

For example, RPE cells can be irradiated using a pulsed dye laser (e.g. by Palomar Medical Inc.) emitting at 590 nm. The pulse energy can be measured using an energy power meter (DigiRad, U.S.A., R-752 Universal Radiometer). During studies that will be described below, ARPE-19 cells were irradiated at various radiant exposures (mJ/cm²) using the experimental set-up shown in Fig. 1. A Helium-Neon laser with an output of 15 mW, coupled into the optical path, was used for the alignment of the laser beam. The pulsed dye laser was coupled to a 1 mm core diameter fiber (Thorlabs, Inc. Newton, N.J., USA, NA = 0.22) and the length of the fiber was 50 m to minimize spatial intensity modulation due to speckle formation at the distal fiber tip. The fiber was directly coupled to a slit lamp fiber.

The fluence can be calculated as described, e.g., in R. Brinkmann, J. Roider, R. Bimgruber, "Selective Retina Therapy (SRT): A review on methods, techniques, preclinical and first clinical results," Bull. Soc. Belge Ophtalmol. 302, 51-60, 2006.

Video images of the laser irradiation region can be captured by a CCD camera through a slit lamp (SL130, Zeiss, Germany), digitized, analyzed, and displayed on a screen or printed. In some experiments described herein, the following parameters were used: a laser fluence of 5 mJ/cm² - 2500 mJ/cm², a laser pulse duration of 1 *µ*s, and 200 spots per 3.5-cm-diameter tissue. Laser treatment spots were separated by, for example, a distance of 2 mm. The laser profile was determined to be about 1.2 mm in diameter. Thus, the laser irradiation was calculated as the pulse energy (measured by the power meter) divided by the spot area.

In some embodiments, laser irradiation can be delivered through a slit-lamp delivery system such that an appropriate radiant exposure is achieved at the focal point of the slit-lamp optics. Neutral density filters can be used for attenuating the laser beams.

In some embodiments, the laser can provide a pulse sequence, for example, at a repetition rate of up to 500 Hz or more. Then, the energy per laser pulse may have lower values to avoid lethal damaging of the tissue during application of a series of laser pulses.

### Methods of Photostimulation

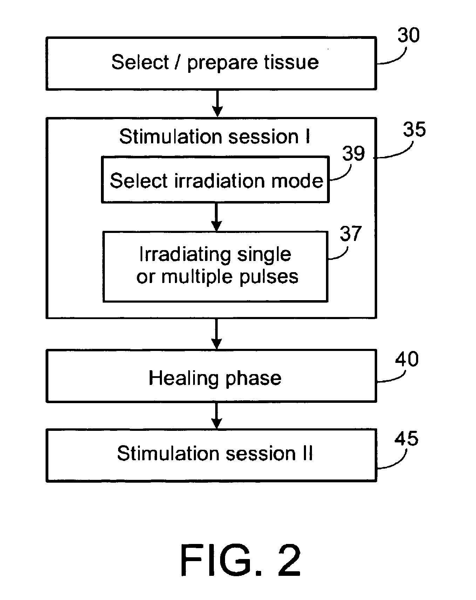

FIG. 2 illustrates a flow diagram of a selective photostimulated treatment protocol. First, one selects a tissue area that one would like to stimulate (step **30).** The tissue area contains either cells showing sufficient pigment concentration or pigment can be introduced before or after the selection (step **30).** The selected area may be diagnosed as being atrophic or the selected area may be related to a damaged area of tissue. In the first case, the goal is to reinstate a healthy or healthier tissue by photostimulated proliferation. In the second case, the goal is to increase the migration rate of healthy cells into the damaged region of tissue.

Next, a first photostimulation treatment session I (step **35)** is performed. During the photostimulation session I, a single or a sequence of laser pulses are irradiated onto the tissue (step **37).** The single laser pulse or the sequence of laser pulses provide a sublethal fluence to the selected area. The irradiation can be performed, for example, in non-moving mode, a continuous-scan mode, or in a pattern mode. If the laser treatment can be executed in multiple modes, a select irradiation step (step **39)** may be part of the photostimulation session I (step **35)** and precede the single or a sequence of laser pulses.

During a so-called "healing" phase (step **40),** the irradiated tissue develops an increased proliferation and tissue repair activity. This phase is the cells' biochemical response to photostimulation. The healing phase may be temporally limited, such that the selective photostimulated treatment includes a second photostimulation session II (step **45)** or a sequence of photostimulation sessions interrupted by healing phases. The number of photostimulation sessions can be determined in advance or can be adjusted during the treatment by observing the success of the treatment. Additionally, the parameter of the photostimulation sessions can vary or can be adopted during the treatment.

The methods described herein involve selectively photostimulating pigmented cells. When an area of tissue containing pigmented cells is irradiated with a laser, such as a q-switched Nd:YAG laser, the cells in the irradiated tissue are induced to proliferate and cause repair and/or regeneration of the tissue. The biological effects are selectively limited to the illuminated region as well as to the cells that express the chromophore that absorbs the laser light. The cells to be targeted may express an endogenous chromophore (e.g., retinal epithelium), or the chromophore can be introduced artificially or by expression of the chromophore within the cells can be induced using a variety of techniques, such as by gene therapy.

In some modes of practicing the invention, a short-pulsed, Nd:YAG q-switched laser is used. Generally, Nd:YAG lasers emit at 1064 nm, and when frequency doubled yield 532 nm output. Both of these wavelengths are useful within the eye, because they are transmitted by ocular media and structures including the cornea, aqueous humor, lens, vitreous, and sclera. In other embodiments, a pulsed dye laser (e.g., a Palomar 3010; 590 nm, 1 µs, 1 mm diameter) can be used, as well as diode pumped solid state lasers, pulsed diode laser systems, or even incoherent pulsed light sources.

In general, the pigment in the cells makes the pigmented target cells optically denser than the nonpigmented surrounding cells, and thus more susceptible to laser-induced photostimulation at selected laser wavelengths and fluences. At sublethal fluences, light, impinging on the target tissue areas for short time durations, selectively stimulates the pigmented target cells with minimal damage to both the target cell and the surrounding cells. The selectivity of tissue stimulation is of great clinical benefit in treating pathological conditions restricted to pigmented cells. For these conditions, the proliferation of the stimulated cells can be increased.

The new methods require the use of laser irradiation that provides a sublethal fluence, or total radiation exposure, for a given treatment session of a specific type of cell with a known or estimated level of endogenous or exogenous pigmentation. At a pulse duration of about 1-5 µs, using a pulsed dye laser or a diode pumped solid state laser, fluences of below 130 mJ/cm², for example, of about 5, 8, 10, 15, 25, 35, 50, 75, 100, 110, or 120 mJ/cm², are sublethal fluences, and are thus effective at photostimulating pigmented RPE cells (with endogenous melanin as the pigment) without causing significant killing of the pigmented cells, and without causing photocoagulation of the tissue. Because of the selective targeting of the new methods, unpigmented cells are also spared, and thus do not mount a cellular response.

At pulse durations of several nanoseconds to several 100 nanoseconds, the sublethal fluence shifts to lower values and can be in the range of from about several µJ/cm² to about several mJ/cm², e.g., about 10 µJ/cm² to 30 mJ/cm², 0.1 to 10, and 1 to 5 mJ/cm². For example, a fluence of less than 10 mJ/cm² for a treatment session of a 10 nanosecond pulse at 532 nm can be a sublethal fluence using a Q-switched Nd-YAG laser. At shorter pulse durations, lower fluences can be effective at photostimulating pigmented RPE cells without causing significant killing of the pigmented cells, and without causing photocoagulation of the tissue. Because of the selective targeting of the new methods, unpigmented cells are also spared for these shorter pulse durations.

The desired radiant exposure can be achieved by modifying the power, target spot size, the beam symmetry, the delivered Joules/pulse, and/or the total number of pulses included in one treatment session. In general, the target spot size is large compared to those utilized in many previous applications of laser therapy to the eye; in some embodiments, the target spot size is from about 0.1 to about 1 mm in diameter. The use of a large target spot size is possible, because the new methods provide selective cell stimulation based on cell pigmentation. A large target area is advantageous: treatment time is minimized when the laser apparatus needs to be redirected fewer times.

Pulse durations of between about 1.0 nanoseconds and about 2.0 µsec, e.g., 50, 100, 250, 500, or 750 nanoseconds, or 1.0 or 1.5 µsec can be utilized. The desired pulse duration is related to the type and size of pigment particle within the target cells to be photostimulated. Because the thermal relaxation of a particle is related to the particle size of the pigment material, smaller intracellular particles require a shorter pulse duration to ensure confinement of energy to the target cells. More specifically, heat is confined within a spherical target for a thermal relaxation time, Tᵣ, which is related to the target diameter, d₂, and the thermal diffusivity constant, D, by Tᵣ = d₂ / 4D. During a lengthy laser exposure, greater than Tᵣ, heat within the target diffuses to surrounding cells or structures. On the other hand, if heat is generated within the target more rapidly than heat can diffuse away, target temperatures become much higher than their surrounding tissues and thermal diffusion to surrounding structures is minimized. Thus, by choosing a pulse duration shorter than the thermal relaxation time of the target pigment (e.g., melanin), selective target stimulation can be achieved. Assuming spherical targets of about 0.5 µm to 5 µm, estimates of thermal relaxation times for biological targets range from 10⁻⁸ s to 10⁻⁶ s.

The emission wavelength of the laser can be within either the visible or infrared spectra (excluding the absorption lines of water for, e.g., ophthalmologic applications). Additional selectivity for target cells is provided by use of an appropriate laser wavelength. For example, when applying the new methods to retinal tissue, incidental absorption by hemoglobin in the retinal vessels may be avoided by selecting a wavelength of 1064 nm for ablating melanin-containing target cells; this wavelength is absorbed by melanin, but not by hemoglobin.

Within the eye, there are several types of pigmented cells, that can be advantageously stimulated when clinically indicated. These cells acquire pigment by either synthesizing melanin endogenously or by phagocytosing exogenous pigment. Cell types that synthesize and retain melanin include the pigmented epithelial cells of the retina, ciliary body, and iris, as well as ocular melanomas.

Although trabecular meshwork (TM) cells are incapable of synthesizing melanin, these cells typically acquire pigment by phagocytosis from the aqueous humor, which normally contains particles of pigmented cellular debris. In addition, in some embodiments, the pigmentation of TM cells can be augmented by adding pigment to the aqueous humor, e.g., by injecting a suspension of pigmented particles into the anterior chamber of the eye with a fine needle. As the aqueous humor with suspended pigment particles flows from the eye through the TM, the TM cells take up pigment, increasing their optical density relative to surrounding nonpigmented tissue, and improving the cell selectivity of the laser stimulation of the cells. In some embodiments, melanin is introduced into the aqueous humor by laser iridotomy of the iris, which releases melanin particles from iris cells into the aqueous humor. Other pigments, such as India ink or any other nontoxic, insoluble particulate dye, can be introduced to phagocytic target cells prior to laser irradiation to stimulate the pigmented cells. In general, the higher the level of pigmentation, the lower the fluence required to achieve photostimulation.

Within the eye, the pigmented target cells may be on the surface of the cornea or conjunctiva, within the cornea or conjunctiva, or may constitute or be attached to any of the intraocular regions of the eye, such as the inner cornea, iris, ciliary body, lens, vitreous, choroid, retina, optic nerve, ocular blood vessels, or sclera. Specifically, diseases and conditions that can be treated by photostimulation include age-related macular degeneration and any other retinal disorders involving the degeneration or death of retinal epithelial cells.

The examples described herein explain the use of photostimulation when regenerating the retinal epithelium in the eye, however, the same approach can be used to promote repair and regeneration of other tissue, such as skin, muscle, or blood vessel walls, or any tissue in which the cells are, and/or can be, selectively pigmented. Thus, the methods described herein are useful for treating any disease or disorder where proliferation and/or migration of pigmented cells is beneficial.

In general, the term treatment is meant to include any photostimulation therapy applied to tissue, e.g., mammalian tissue, without causing photocoagulation and without causing significant damage to, or cell death in, the treated tissue. Patients who can be treated by the new methods described herein include humans, as well as nonhuman primates, sheep, horses, cattle, goats, pigs, dogs, cats, rabbits, guinea pigs, hamsters, gerbils, rats, and mice.

### Methods of Treating Macular Degeneration

FIG. 2 illustrates steps in the new methods of selective photostimulation, e.g., as applied to treat diseases of the eye, e.g., for macular degeneration, specifically, age-related macular degeneration. The selection of the tissue area to be irradiated is based on the condition of the degenerated retinal pigment epithelium of the macular, which can be evaluated at the beginning of the treatment session using a conventional slit lamp. Then, the selected tissue area is either manually or automatically irradiated with light of the appropriate parameters, e.g., laser light of a wavelength of 590 nm, a pulse duration of about 1-5 µs, and laser fluence of less than 130 mJ/cm².

The temporal separation between successive pulses in a sequence of pulses can affect the sublethal fluence. For example, a short temporal separation may cause an accumulative effect in the interaction of the pulses with the cells, thereby reduce the sublethal fluence. In some embodiments, pulse sequences include, for example, 10-100 pulses at a repetition rates of about 500 Hz (e.g., pulse separations from below 1 ms to several hundred ms). The healing phase, for example, between treatment sessions, can extend over one or more hours, one day, a few days, or a few weeks.

### Methods of Wound Healing

Selective photostimulation therapy can be also applied to heal wounds of various tissues. Specifically, if tissue containing (or treated to contain) a particular pigment or chromophore is damaged, increased tissue repair can be induced by sublethal photostimulation. Wounds can be caused, for example, by mechanical interaction with the tissue during a surgery or by some trauma, e.g., an accident. Then, the selection of the tissue area can be based on the extent of the damaged tissue. The selected tissue area is either manually or automatically irradiated with laser light of the appropriate parameters.

### Methods of Combined Photocoagulation Followed by Photostimulation

A specific example of laser induced damage of tissue in the eye is laser surgery, for example, laser coagulation of the eye. As a side effect of laser surgery, areas of, for example, the retina, can be damaged. To promote healing and to increase the migration of healthy tissue from neighboring areas, selective photostimulation can be applied in connection with the laser surgery. For example, either immediately after photocoagulation, or in a later session, e.g., a few hours or one or more days or a week, after the laser surgery, the coagulated tissue and the areas of tissue adjoining the coagulated tissue are irradiated in a separate treatment session with sublethal fluence to increase the proliferation and migration of the epithelium and speed healing after the surgery.

### EXAMPLES

The invention is further described in the following examples, which do not limit the scope of the invention described in the claims.

### Materials and Experimental Methods

In the following experiments, laser irradiation parameters (using a Palomar Medical Inc. pulsed dye laser emitting at 590 nm with a 1 mm diameter) included: 5.0 mJ/cm² - 2550 mJ/cm² of laser radiant exposure, 1 *µ*s duration, and 200 spots per 3.5-cm-diameter dish. Laser exposure spots were separated by a distance of 2 mm. Four sample dishes were irradiated per experimental condition. The laser radiant exposure was calculated as the pulse energy measured by a power meter divided by the spot area.

The results are presented as a mean ± S.D., and n states the number of samples. Statistical comparisons were made using Student's t-tests, and p ≤0.05 was considered statistically significant.

Adult ARPE-19 cell cultures were artificially pigmented by incubation with melanin. The cells were then irradiated at various laser radiant exposures using a single 1 *µ*s pulse dye laser tuned to 590 nm. The viability of the pigmented adult ARPE-19 cells was evaluated using a fluorescent live/dead cytotoxicity assay. In vitro wound healing and cell regeneration models were used to study the subsequent photo/thermal stimulation effects of laser irradiation. The expressions of platelet-derived growth factor (PDGF) and its receptor by the adult ARPE-19 cells in response to laser irradiation were evaluated by the reverse transcription-polymerase chain reaction (RT-PCR).

In general, the following phenomena were observed: (1) selective targeting of pigmented RPE cells produced no thermal damages to adjacent non-pigmented cells even at laser radiant exposure of 2550 mJ/cm²; (2) exposure to laser irradiation (< 120 mJ/cm²) enhanced RPE cell proliferation and migration; and (3) the selective photostimulation increased the expressions of PDGF, TGFβ1, bFGF, EGF, IGF, and their receptors, VEGF, PEDF, and heat stock proteins after laser irradiation.

Thus, these experiments demonstrate the selective treatment of pigmented RPE cells in vitro, as well as photo/thermal stimulation of RPE proliferation and related biological mechanisms. These results can be applied in a treatment modality for RPE related retinal disorder diseases such as age-related macular degeneration. The experiments show a temporal variation in growth factor expression in RPE following selective photostimulation which infers that RPE wound healing after selective photostimulation is regulated by growth factors in an autocrine manner and these growth factors work in harmony to elicit wound repair.

### Reagents

Dulbecco's modified Eagle medium/F12 (DMEM/F12), fetal bovine serum (FBS), phosphate-buffered saline (PBS), 100X penicillin-streptomycin stock solution, and 0.5% trypsin-0.02%EDTA stock solution were obtained from Invitrogen Life Technologies (Carlsbad, CA). F-12K Medium (Kaighn's Modification of Ham's F12) was purchase from ATCC (Manasses, VA). Sepia melanin, urea, thiourea, CHAPS, DTE, EGTA, and EDTA were obtained from Sigma-Aldrich Chemicals (St. Louis, MO). Protease inhibitors were purchased from Roch Applied Science (Indianapolis, IN). Bradford protein assay reagent was obtained from Bio-Rad Laboratories (Hercules, CA). Fluorescent live/dead viability/cytotoxicity assay was obtained from Molecular Probes

(Eugene, OR, U.S.A.). ELISA kit for platelet-derived growth factor (PDGF)-BB was purchased from R&D Systems Inc. (Minneapolis, MN). Nuclearspin® RNA II kit was obtained from Clontech Laboratories, Inc. (Mountain View, CA). SuperScriptTM III First Strand kit was purchased from Invitrogen Life Technologies (Carlsbad, CA) and PCR core system was purchased from Promega (Madison, WI). Microcon Centrifugal filter devices with molecular weight cut off 3000 Dalton was obtained from Millipore (Billerica, MA).

### Collection of Conditioned Media

Pigmented ARPE-19 cells grew to post-confluence to gain quiescence. Pigmented ARPE-19 cells were irradiated by pulsed dye laser at different laser fluences in PBS. Immediately after laser irradiation, cell culture medium with 1% FBS was used to replace PBS. Irradiated cells were incubated at 37°C 5% CO₂. Conditioned medium was collected 48 hours after laser irradiation.

### Example 1 - Enhancing, Melanin Content in Cells

### Mixed Non-Pigmented and Pigmented ARPE-19 Cells

Human ARPE-19 cells were grown in two matching flasks (75 cm²) to confluence as described above. One of the flasks was fed with melanin for 20 hours, and subsequently washed with PBS. The ARPE-19 cell cultures (non-pigmented and pigmented) were then trypsinized (0.5% trypsin-0.02%EDTA) for 10 minutes. The ARPE-19 cell suspensions were recovered by centrifugation (500 rpm for 5 minutes), and re-suspended in medium. The two cell suspensions were combined and thoroughly mixed, yielding a suspension of a 1:1 mixture of non-pigmented cells and pigmented cells. The mixed cell suspension was immediately re-plated in a 35 mm dish at the confluent density, incubated for 24 hours to allow the formation of a continuous cell sheet, and then irradiated.

### Assessment of Melanin Content in Pigmented RPE Cells

Cultured cells were detached with 1 ml of 0.25% trypsin with 0.02% EDTA (10 minutes at 37°C). An aliquot (50 *µ*l) was removed, and cells were counted using a hemocytometer. The remaining cell suspension was centrifuged, and the pellet was dissolved in 1 N NaOH. The melanin concentration was determined by measurement of absorption at 475 nm, and then compared with a standard curve obtained using synthetic melanin.

### Example 2 - Selective Photostimulation of RPE Cells

### RPE Cell Culture and Phagocytosis of Melanin

The experiments described herein were performed using third, fourth and fifth passage human retinal pigment epithelial (ARPE-19) cells. ARPE-19 cells (obtained from ATCC) were cultured in Dulbecco's modified Eagle's medium/F12 (1:1) with 10% fetal bovine serum, 1% penicillin-streptomycin at 37°C, in a 5% CO₂ balance air atmosphere. The cells were cultured in tissue culture treated culture dishes of 3.5 cm in diameter. The ARPE-19 cells are non-pigmented in their normal growth state and serve as the control non-pigmented RPE cells.

To obtain pigmented ARPE-19 cells, confluent ARPE-19 cell cultures were incubated for 20 hours with varying concentrations of sepia melanin as previously described. Prior to the incubation, sepia melanin was washed with RPE cell culture medium and sonicated to obtain a uniform suspension. Just prior to the irradiation, the medium was replaced with PBS to avoid absorption of laser energy by the medium, and the PBS was replaced with standard medium following laser irradiation.

### Selective Targeting of Pigmented Cells

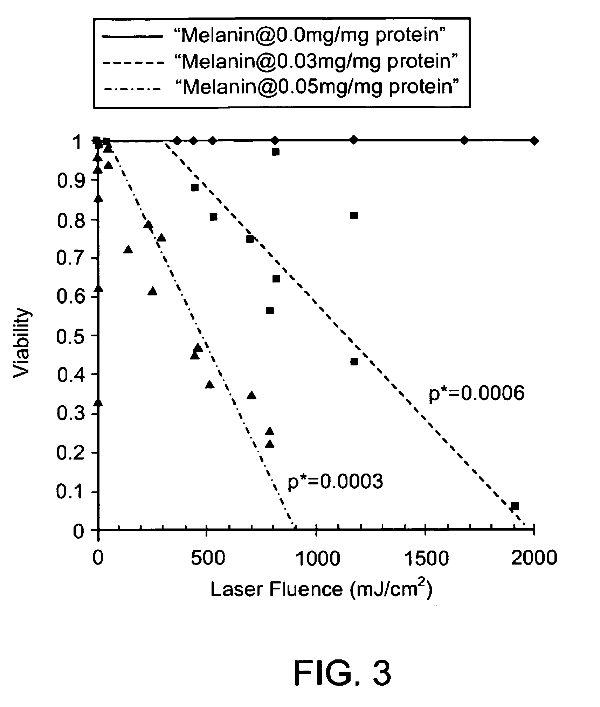

Although non-pigmented cells appear not to absorb the laser irradiation, thermal energy absorbed by pigmented cells could potentially damage their neighboring non-pigmented cells. In FIG. 3, the fitting curves represent the least-square linear fits of the experimental data with the following regression parameters (a=slope; b=ordinate; threshold=intersection with 100% viability line; r=regression coefficient); Melanin content 0.03 mg/mg protein: a=0.99; b=-9.8×10-4; r=0.98; threshold=1140 mJ/cm²; Melanin content 0.05 mg/mg protein: a=1.05; b=-1.1×10-3; r=0.98; threshold=460 mJ/cm2; (y=ax+b); P values shown are comparisons with non-pigmented cells.

To assess the selectivity of laser treatment, its effects on mixed cell populations of pigmented and non-pigmented ARPE-19 cells were analyzed using a high laser radiant exposure (1100 mJ/cm²). This laser radiant exposure corresponds to 50% viability for RPE cells preincubated in 0.03 mg/mg protein melanin content. The selective targeting results of pigmented ARPE-19 cells did not show any associated damage to their adjacent non-pigmented cells. Only the ARPE-19 cells within the irradiation zone containing melanin were selectively killed, whereas the adjacent non-pigmented ARPE-19 cells showed no evidence of cellular damage. The viability of pigmented ARPE-19 cells was around 50%, which was significantly lower than that of non-pigmented ARPE-19 cells, which was 100% (p < 0.001, n = 6). Immediately following the laser irradiation, the damage to the ARPE-19 cells containing pigments was so subtle that by phase contrast microscopy alone, it was difficult to morphologically differentiate affected from non-affected cells.

These results support that melanin containing ARPE-19 cells can be selectively targeted without significant collateral damage to the adjacent non-pigmented ARPE-19 cells at the laser radiant exposure levels up to about 1100 mJ/cm².

### Example 3 - Cell Viability and Proliferation Assays

ARPE-19 cells were seeded onto 96-well plates at a density of 1000 cells/well in conditioned medium collected 48 hours after laser irradiation. The cell culture medium was changed twice a week. Cell proliferation was assessed by colorimetric dimethylthiazol diphenyl tetrazolium bromide (MTT) assay. For analysis the culture medium was removed and 100 µl of PBS containing 10 µL of MTT (5 mg/ml) was added to each well. The cells were then incubated at 37°C for 3 hours. The reaction was terminated by adding 0.04 N HCL in isopropanol. The absorbance of the medium was then determined using an ELISA reader in 570 and 630 nm. The differences between the absorbance at the two wavelengths served as an estimate for cell viability. Individual experiments were repeated at least three times by using three replicates for each experiment.

### Cellular Viability of Pigmented RPE Cells Following Laser Irradiation

The viability of pigmented ARPE-19 cells was evaluated using a fluorescent live/dead viability/cytotoxicity assay. The fluorescent live/dead viability/cytotoxicity assay utilizes ethidium homodimer and calcein-AM, which localize to dead and live cells, respectively. The assay solutions were prepared according to the manufacturer's recommended protocol. Briefly, 200 *µ*l of the solution (2.0 *µ*M calcein-AM and 4.0 *µ*M ethidium homodimer-1 in PBS) were applied to each cell culture dish. The culture was incubated for 20 minutes at 37°C, and then analyzed using fluorescence microscopy (Zeiss Axiovert 200M, Carl Zeiss MicroImaging, Inc.).

Live cells were distinguished by the presence of ubiquitous intracellular esterase activity, determined by the enzymatic conversion of the virtually nonfluorescent cell-permeant calcein-AM to the intensely fluorescent calcein. The polyanionic calcein dye is well retained within live cells, producing an intense uniform green fluorescence (excitation/emission ∼495 nm/515 nm). Ethidium homodimer-1 (EthD-1) enters the cells with damaged membranes and undergoes a 40-fold enhancement of fluorescence upon binding to nucleic acids, thereby producing bright red fluorescence in dead cells (excitation/emission ∼ 495 nm/635 nm). EthD-1 is excluded by the intact plasma membrane of live cells. Cellular viability was measured as an average of 3 irradiated spots (440 µm diameter) divided by non-irradiated spots on the same dish. Images were taken at the identical gain and exposure settings.

Referring to FIG. 3, at a laser radiant exposure of 5 mJ/cm², the cytoplasmic staining demonstrated that there was no obvious cellular injury on pigmented RPE cells. When the laser radiant exposure was increased to 30 mJ/cm², a few dead cells with the red nuclear staining appeared. With the increasing laser radiant exposure, more and more dead cells with the red nuclear staining were observed. At the highest laser radiant exposure of 2550 mJ/cm² all the cells with a melanin level of 0.03 mg/mg protein (■) and within the laser spot size were damaged or killed. For the sham controls, consisting of irradiated cells without melanin (top trace in FIG. 3 (◆)), the fluorescent cytotoxicity assay showed no evidence of cellular injury using radiant exposure even at 2550 mJ/cm². Cells with the highest melanine concentration of 0.05 mg/mg protein (A) were all dead at a fluence of about 900 mJ/cm².

For the viability quantification of the pigmented RPE cells following laser irradiation, the objective area within a radius of laser spot size was used to count the live /dead cells to evaluate cellular viability as a function of laser radiant exposure as well as melanin content. The viability of pigmented ARPE-19 cells was defined as the green fluorescent intensity ratio. This ratio was calculated by the green fluorescent intensity within the laser spot size after laser irradiation, divided by that within a similar spot size without laser irradiation. The cellular viability as a function of laser radiant exposure and melanin content of the RPE cells was demonstrated.

In general, the higher the laser radiant exposure, the more pigmented cells were targeted. With the increase of melanin content, this critical range of laser radiant exposure decreased. Therefore, the melanin content played a significant role during laser radiation.

### BrdU Assay

Pigmented ARPE-19 cells were seeded in 35 mm dishes in serum free medium F-12K medium for 24 hours. Cells were irradiated in PBS. PBS was supplemented with cell culture medium after laser irradiation. Six hours later, 10 µM BrdU was added to cell culture medium and incubated overnight at 37°C. The cells were fixed with 70% ethanol for 45 minutes at room temperature, and incubated with 4M HCL for 20 minutes at room temperature. Then, cells were permeabilized with 0.2% Tritox X-100® for 15 minutes at room temperature, and genomic DNA was denatured by adding 1 ml/dish of blocking buffer (PBS/10%FBS) for 10 minutes at room temperature. 10 µl anti-BrdU-Alex594 stock solutions were added and incubated for 60 minutes at 37°C. Then the cells were washed three times. Fluorescence intensity was evaluated by fluorescence microscopy at wavelength of 594 nm. The fluorescence intensities of laser irradiated samples are quantified by METAMORPH® software and normalized by fluorescence intensity of sham control.

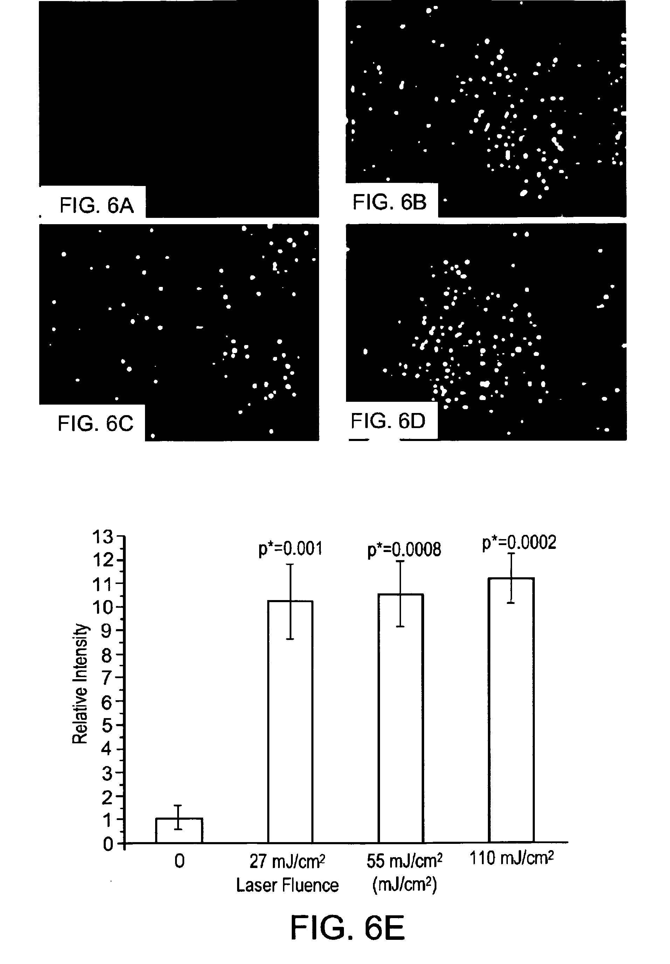

To determine the proliferation response of pigmented RPE cells to irradiation, we quantified BrdU incorporation 6 hours after laser irradiation. BrdU staining demonstrated a significantly enhanced proliferation of pigmented RPE cells exposed to sublethal laser irradiation as shown in FIGs. 6A-6E. FIG. 6A shows a Sham Control image. Further, laser irradiated samples at a laser fluence of 28 mJ/cm² (FIG. 6B), at 55 mJ/cm² (FIG.6C), and at 110 mJ/cm² (FIG. 6D). The scale bar is 200 *µ*m. Comparisons of p values refer to Sham Control N=3.

### Example 4 - Cell Regeneration Assays

### Cell Regeneration Following Laser Irradiation

An amount of 1.0×10⁶ ARPE-19 cells were seeded, grown to confluence where there is minimal proliferation, and fed with melanin as described above. The cells were irradiated in PBS. Immediately after irradiation, the medium with 1% FBS was used to replace PBS. The low percentage serum was used to constrain cell proliferations. The conditioned-medium was collected 48 hours after laser irradiation. After three laser irradiation sessions, the medium with 20% FBS was used to follow the process of laser-induced cell regeneration. The controls were handled similarly without laser irradiation. The images of the view fields were acquired at 10X magnification using an inverted microscope (Axiovert® 200 M, Zeiss, USA) with a digital camera (AxioCam® MRm, Zeiss, USA). The cell numbers during in vitro cell regenerations were quantified manually for each view field. Three fields were randomly picked for quantification.

### RPE Cell Regeneration Following Laser Irradiation

Following laser irradiation, PBS was replaced with the cell culture medium containing 1% FBS. Then the cells were exposed to the laser irradiation treatment every other day for a total of three treatments. On Day 6, the cell culture medium was supplemented with 20% FBS and the cells were cultured for additional 11 days (From Day 6 to Day 17) to follow cell regeneration. ARPE-19 cells undergoing this procedure, without laser treatment (sham control) demonstrated poor regeneration following 11 days of serum treatment. On the other hand, the ARPE-19 cells exposed to low power laser irradiation demonstrated good regeneration after changing to a cell culture medium with 20% FBS.

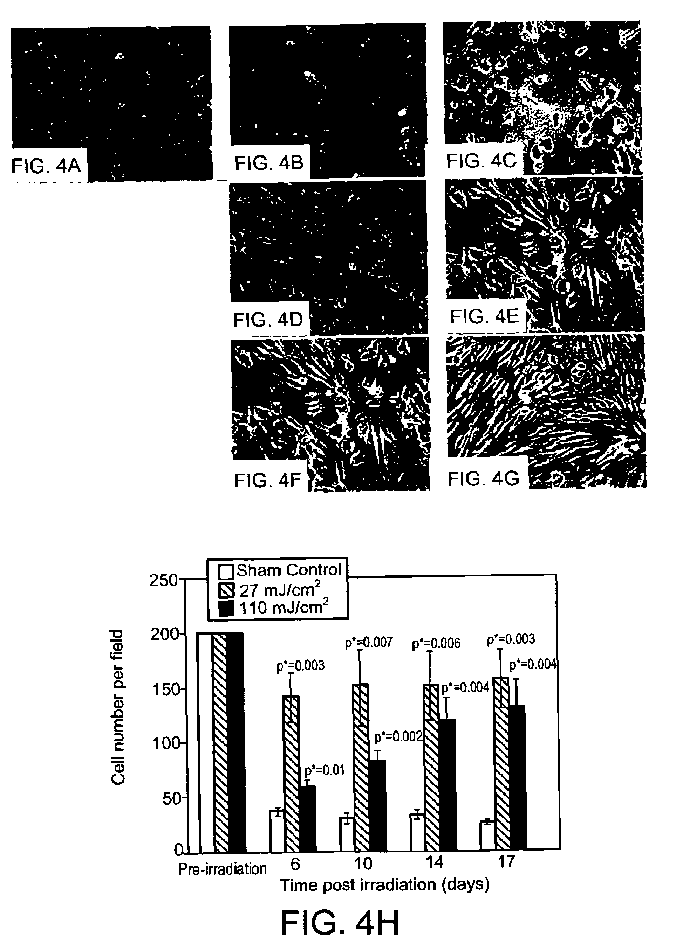

FIGs. 4A to 4H illustrate RPE cell regeneration following laser irradiation. FIG. 4A shows confluent cells prior to laser irradiation. FIGS. 4B through 4G show the phase images of ARPE-19 cells in the sham controls, following three laser irradiation sessions, and the subsequent regenerations in the cell culture medium with 20% FBS after laser irradiation. In particular, FIG. 4A shows a phase image of confluent ARPE-19 cells prior to laser irradiation; FIG. 4B shows a Sham Control image on day 6; FIG. 4C shows a Sham Control image on day 17; FIG. 4D shows an image of ARPE-19 cells irradiated at 27 mJ/cm² on day 6; FIG. 4E shows an image of ARPE-19 cells irradiated at 27 mJ/cm² on day 17. FIG. 4F shows an image of ARPE-19 cells irradiated at 110 mJ/cm² on day 6; FIG. 4G shows an image of ARPE-19 cells irradiated at 110 mJ/cm² on day 17.

FIG. 4H illustrates cell number per field as function of time. The scale bar is 100 *µ*m, and p values are for comparisons with sham controls. Specifically, FIG. 4H shows the cell numbers as a function of laser radiant exposures and day during in vitro cell regeneration experiments. For the sham control, the cell number dropped significantly to less than 50 per view field on Day 6, and did not increase significantly 11 days after changing the medium to 20% FBS. This decrease likely reflected the cytotoxicity of endocytosed melanin, which had also been reported previously. The laser irradiated samples at the laser radiant exposure of 27 mJ/cm² appeared good morphologically on Day 6, and cell number did not drop significantly afterwards (p = 0.003 compared to the sham controls, n = 3). For the laser irradiated samples at the laser radiant exposure of 110 mJ/cm², the cell number initially dropped, but then increased significantly until Day 17 after changing to the 20% FBS-supplemented culture medium (p = 0.004 compared to the sham controls, n = 3).

### Example 5 - Wound Healing Assays

### In Vitro Wound Healing Assay

Pigmented ARPE-19 cells were cultured to post-confluence, where there is minimal proliferation, as previously described. To simulate a wound environment, confluent monolayers were gently stroked using a 1 ml pipette tip. This process makes a uniform lane that is devoid of cells. The cells were then incubated at 37°C for 30 minutes before being irradiated. The culture medium was replaced by PBS for the duration of the laser irradiation. Following the laser irradiation, PBS was replaced by culture medium with 1% FBS. The purpose of switching FBS from 10% to 1% is to constrain RPE cell proliferation. The medium was replaced every 48 hours, and the cultures were subjected to laser irradiation every 48 hours. Changes in normal and wounded ARPE-19 cell morphologies were evaluated by light microscopy. The cell number was counted during the process.

### Sublethal Laser Irradiation Promotes Wound Healing Response

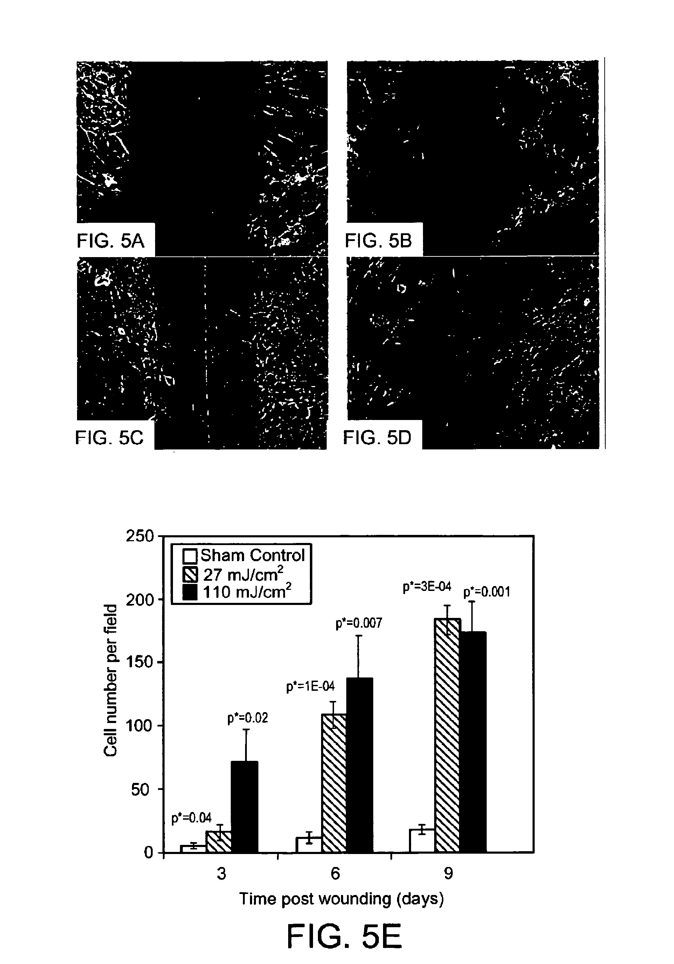

Damage to the RPE cell layer is an important part of macular degeneration. Here the attempt was made to discern whether sublethal laser irradiation can stimulate the RPE recovery following injury. With a standard scratch assay model, the healing response of irradiated and non-irradiated cells were compared as shown in FIGs. 5A through 5E.

FIGs. 5A to 5E shows phase images of in vitro wound healing. In particular, FIG. 5A shows a scratch model prior to laser irradiation. FIG. 5B shows a Sham Control image 3 days following wound injury. FIG. 5C shows cell cultures irradiated at 27 mJ/cm² after 3 days of wound injury. FIG. 5D shows a cell culture irradiated at 110 mJ/cm² after 3 days of wound injury. FIG. 5E shows cell number per field (the field is in the middle of wound, the area of wound is about 2 mm²) as a function of day. The scale bar is 100 *µ*m, and p value refer to comparisons with respective sham controls.

The cell number as a function of laser radiant exposure and day during the in vitro wound healing assay are represented in FIG. 5E. The scar margins remained clearly defined in the non-irradiated sham control (FIG. 5B) even after 3 days of culturing. In contrast, the pigmented ARPE-19 cells that were treated with laser irradiation at energy levels varying from 27 mJ/cm² to 110 mJ/cm² showed an increase in cell density (p = 0.001 compared to the sham controls, n = 3) around the wound margin and displayed a greater rate of cell migration and proliferation across the wound in an attempt to close the wound region (FIGS. 5C and 5D). However, the non pigmented cells were unaffected by laser irradiation (data not shown).

### Example 6 - Growth Factor Stimulation Assay

### RNA Isolation and RT-PCR

ARPE-19 cells were grown to post-confluence, where there is minimal proliferation, as mentioned earlier. The pigmented ARPE-19 cells were seeded in 35 mm dishes in the serum free medium F-12K medium for 24 hours. The cells were irradiated in PBS. The PBS was supplemented with F-12K after laser irradiation. The total RNA was extracted from the cultured pigmented ARPE-19 cells 8 hours after laser irradiation according to the manufacturer's protocol. Reverse transcription was conducted on 10 ng total RNA using the First Strand SUPERSCRIPT® Preamplification System for First Strand cDNA Synthesis with oligo(dT)20 primers according to the protocol of the manufacturer. Five microliters of cDNA were added to the PCR mixture in a final volume of 50 µl containing 50 mM KCI, 10 mM Tri-HCl, 2.5 mM MgCl₂, and 10 mM each of dNTPs and 1U heat-stable DNA polymerase from *Thermus brockianus* (Promega, USA). The following PCR cycle parameters were used for 40 cycles: initial denaturation for 2 minutes at 95°C, annealing at 55°C for 1 minutes; polymerization at 72°C for 1 minutes; followed by a final extension at 72°C for 2 minutes.

The parallel RT-PCR reactions without reverse transcriptases were performed for each sample to confirm that the PCR products resulted from cDNA rather than from genomic DNA. β-action was used as a constitutively expressed gene product for the comparison of PDGF and PDGF receptor mRNA abundance between samples. All products were then analyzed with 2% agarose gel electrophoresis and ethidium bromide staining, and the resulting bands were densitometrically scanned. The specific 5' and 3' primers for PDGF-A, PDGF-B, PDGFR-α, and PDGFR-β were obtained commercially.

### Expression of PDGF and its Receptor mRNA in Pigmented RPE Cells

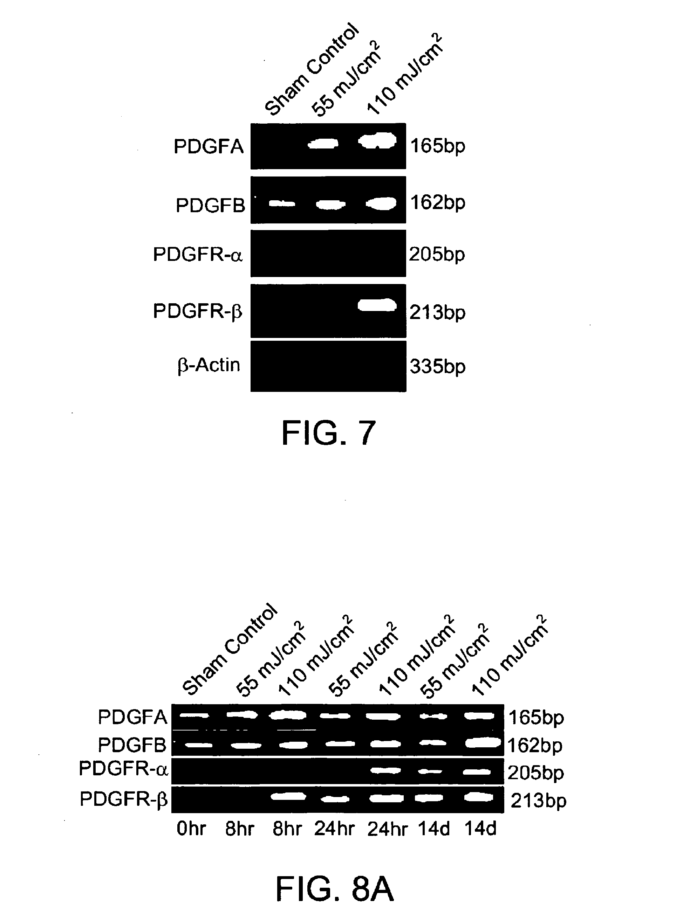

The mRNA levels of PDGF-A and B chains, and PDGF receptors α and *β* in pigmented ARPE-19 cells with and without laser irradiation were evaluated. The total RNA extracted from cells for 8 hours after such treatments was used for the determination of mRNA levels by RT-PCR analysis. ARPE-19 cells constitutively expressed PDGF-A and B, and receptors α and *β* mRNA as illustrated in FIG. 7, which shows a RT-PCR analysis of PDGFA, PDGFB, PDGFR-α, PDGFR-*β* in sham control and laser irradiated RPE cells.

PDGF-A, B, and receptors α and *β* expressions were increased by laser irradiation at 55 mJ/cm² and 110 mJ/cm² compared to the sham controls. The identity of reaction products of expected size was confirmed by agarose gel electrophoresis. No PCR products were obtained in the absence of reverse transcriptase, indicating that RNA rather than genomic DNA, served as the template.

### Gene Expression of Growth Factors, Their Receptors, and Heat Shock Proteins

The expression of PDGFA, PDGFB, and their receptors were up-regulated after selective photocoagulation. The level of PDGFA was 1.35±0.005 (P = 4.57E-08, n = 3) (laser fluence of 55 mJ/cm²) and 2.11 ± 0.09 (P = 6.92E-05, n = 3) (laser fluence of 110 mJ/cm²) times the sham control level at 8 hours, then decreased to 1.12 ± 0.04 (p = 0.028, n = 3) (laser fluence of 55 mJ/cm²) and 1.41 ± 0.04 (p = 0.0008) (laser fluence of 110 mJ/cm²) times the sham control level at 24 hours, to 1.12±0.13 (p = 0.28, n = 3) (laser fluence of 55 mJ/cm²) and 1.28 ± 0.22 (p = 0.15, n = 3) (laser fluence of 110 mJ/cm²) times the sham control level at 2 weeks after laser photocoagulation.

The expression level of PDGFα receptor was 1.08±0.03 (P=0.044, n=3) (laser fluence of 55 mJ/cm²) and 1.60±0.17 (P=0.033, n=3) (laser fluence of 110 mJ/cm²) times the sham control level at 8 hours, then decreased to 1.31±0.08 (P=0.005, n=3) (laser fluence of 55 mJ/cm²) and 1.76±0.03 (P=5.39E-06, n=3) (laser fluence of 110 mJ/cm²) times the sham control level at 24 hr, to 1.22±0.22 (P=0.21, n=3) (laser fluence of 55 mJ/cm²) and 1.73±0.03 (P=5.96E-06, n=3) (laser fluence of 110 mJ/cm²) times the sham control level at 2 weeks after laser photocoagulation.

The level of PDGFB was 1.22±0.02 (P=4.94E-05, n=3) (laser fluence of 55 mJ/cm2) and 1.44±0.11 (P=0.005, n=3) (laser fluence of 110 mJ/cm2) times the sham control level at 8 hours, then increase a little bit to 1.22±0.02 (P=0.0009, n=3) (laser fluence of 55 mJ/cm2) and 1.45±0.03 (P=0.0001) (laser fluence of 110 mJ/cm2) times the sham control level at 24 hours, to 1.24±0.32 (P=0.28, n=3) (laser fluence of 55 mJ/cm²) and 1.85±0.05 (P=1.38E-05, n=3) (laser fluence of 110 mJ/cm2) times the sham control level at 2 weeks after laser photostimulation.

The expression level of PDGF*β* receptor was 1.28±0.03 (P=9.65E-05, n=3) (laser fluence of 55 mJ/cm²) and 1.60±0.06 (P=0.033, n=3) (laser fluence of 110 mJ/cm²) times the sham control level at 8 hours, then increased to 1.80±0.17 (P=0.003, n=3) (laser fluence of 55 mJ/ cm²) and 2.97±0.19 (P=0.001, n=3) (laser fluence of 110 mJ/cm²) times the sham control level at 24 hr, to 2.04±0.2 (P=0.002, n=3) (laser fluence of 55 mJ/cm²) and 3.00±0.03 (P=1.87E-07, n=3) (laser fluence of 110 mJ/ cm²) times the sham control level at 2 weeks after laser photostimulation. (FIG. 8A)

The expression level of TGF-*β*1 was 1.40±0.29 (P=0.116, n=3) (laser fluence of 55 mJ/cm²) and 2.38±0.11 (P=6.67E-05, n=3) (laser fluence of 110 mJ/cm²) times the sham control level at 6 hours, then decreased to 1.07±0.01 (P=0.002, n=3) (laser fluence of 55 mJ/cm²) and 1.85±0.05 (P=0.0001) (laser fluence of 110 mJ/cm²) times the sham control level at 24 hours, to 1.11±0.06 (P=0.06, n=3) (laser fluence of 55 mJ/cm²) and 1.31±0.07 (P=0.004, n=3) (laser fluence of 110 mJ/ cm²) times the sham control level at 2 weeks after laser photostimulation.

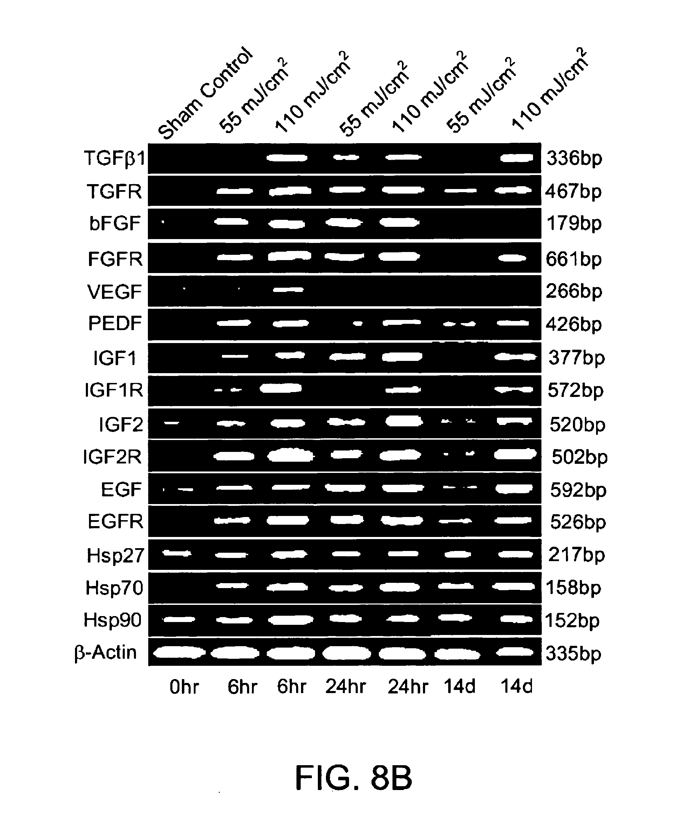

The expression level of TGF receptor was 1.53±0.15 (P=0.0075, n=3) (laser fluence of 55 mJ/cm²) and 2.05±0.09 (P=6.39E-05, n=3) (laser fluence of 110 mJ/ cm²) times the sham control level at 6 hours, then decreased to 1.1±0.14 (P=0.4, n=3) (laser fluence of 55 mJ/cm²) and 2.02±0.08 (P=5.61E-05, n=3) (laser fluence of 110 mJ/cm²) times the sham control level at 24 hours, to 1.85±0.05 (P=0.37, n=3) (laser fluence of 55 mJ/cm²) and 1.5±0.11 (P=0.003, n=3) (laser fluence of 110 mJ/cm²) times the sham control level at 2 weeks after laser photostimulation. (FIG 8B).

The expression of bFGF was 1.54±0.23 (P=0.029, n=3) (laser fluence of 55 mJ/ cm²) and 1.93±0.18 (P=0.002, n=3) (laser fluence of 110 mJ/ cm²)times the sham control level at 6 hours, then decreased to 1.08±0.02 (P=0.003, n=3) (laser fluence of 55 mJ/ cm²) and 1.23±0.02 (P=0.0001, n=3) (laser fluence of 110 mJ/ cm²) times the sham control level at 24 hours, to 1.0±0.04 (P=0.725, n=3) (laser fluence of 55 mJ/cm²) and 1.04±0.03 (P=0.191, n=3) (laser fluence of 110 mJ/cm²) times the sham control level at 2 weeks after laser photostimulation.

The expression level of FGF receptor was 1.73±0.015 (P=0.00043) (laser fluence of 55 mJ/ cm²) and 2.72±0.42 (P=0.055) (laser fluence of 110 mJ/cm²) times the sham control level at 6 hours, then decreased to 1.27±0.01 (P=2.11E-06, n=3) (laser fluence of 55 mJ/cm²) and 1.46±0.12 (P=0.006, n=3) (laser fluence of 110 mJ/cm²) times the sham control level at 24 hours, to 1.0±0.035 (P=0.42, n=3) (laser fluence of 55 mJ/cm²) and 1.24±0.01 (P=0.0017, n=3) (laser fluence of 110 mJ/cm²) times the sham control level at 2 weeks after laser photostimulation (FIG 8B).

The gene expression of IGF-1 had also increased by 6 hours, then decreased at 24 hours till 2 weeks. Thus, the level was 1.39±0.10 (P=0.005, n=3) (laser fluence of 55 mJ/cm²) and 1.86±0.18 (P=0.0027, n=3) (laser fluence of 110 mJ/cm²) times the sham control level at 6 hours, then decreased to 1.25±0.18 (P=0.125, n=3) (laser fluence of 55 mJ/cm²) and 1.56±0.85 (P=0.097, n=3) (laser fluence of 110 mJ/cm²) times the sham control level at 24 hours, to 1.0±0.04 (P=1, n=3) (laser fluence of 55 mJ/cm²) and 1.31±0.17 (P=0.061, n=3) (laser fluence of 110 mJ/cm²) times the sham control level at 2 weeks after laser photostimulation.

The expression level of IGF1 receptor was 1.22±0.11 (P=0.044, n=3) (laser fluence of 55 mJ/cm²) and 2.27±0.24 (P=0.0016, n=3) times the sham control level, decreased to 1.05±0.13 (P=0.75, n=3) (laser fluence of 55 mJ/cm²) and 1.51±0.12 (P=0.048, n=3) (laser fluence of 110 mJ/cm²) times the sham control level at 24 hours, then decreased to 1.0±0.04 (P=1, n=3) (laser fluence of 55 mJ/ cm²) and 1.18±0.16 (P=0.193, n=3) (laser fluence of 110 mJ/cm²) times the sham control level at 2 weeks after laser photostimulation. (FIG 8B)

The gene expression of IGF-2 had also increased by 6 hours, then decreased at 24 hours and remained nearly constantly at 2 weeks. Thus, the level was 1.27±0.12 (P=0.033, n=3) (laser fluence of 55 mJ/cm²) and 1.73±0.09 (P=0.00039, n=3) (laser fluence of 110 mJ/cm²) times the sham control level at 6 hours then decreased to 1.13±0.06 (P=0.106, n=3) (laser fluence of 55 mJ/cm²) and 1.65±0.10 (P=2.23E-07, n=3) (laser fluence of 110 mJ/ cm²)times at 24 hours, to 1.07±0.07 (P=0.212, n=3) (laser fluence of 55 mJ/cm²) and 1.21±0.05 (P=0.004, n=3) (laser fluence of 110 mJ/cm²) times at 2 weeks after laser photostimulation.

The expression level of IGF2 receptor was 1.39±0.22 (P=0.064, n=3) (laser fluence of 55 mJ/cm²) and 2.62±0.21 (P=0.006, n=3), decreased to 1.29±0.05 (P=0.024, n=3) (laser fluence of 55 mJ/ cm²) and 1.89±0.10 (P=0.011, n=3) (laser fluence of 110 mJ/ cm²) times the sham control level at 24 hours then decreased 1.0±0.02 (P=0.71, n=3) (laser fluence of 55 mJ/ cm²) and 1.41±0.32 (P=0.142, n=3) (laser fluence of 110 mJ/ cm²) at 2 weeks after laser photostimulation. (FIG. 8B)

The gene expression of EGF had increased by 6 hours, 24 hours then decreased at two weeks. Thus, the level was 1.17±0.06 (P=0.014, n=3) (laser fluence of 55 mJ/ cm2) and 1.53±0.07 (P=0.00042, n=3) (laser fluence of 110 mJ/ cm2) times the sham control level at 6 hours, then increased to 1.57±0.37 (P=0.092, n=3) (laser fluence of 55 mJ/ cm²) and 2.45±0.14 (P=0.00012, n=3) (laser fluence of 110 mJ/ cm²) times the sham control level at 24 hours, to 1.07±0.05 (P=0.131, n=3) (laser fluence of 55 mJ/ cm²) and 2.17±0.39 (P=0.0014, n=3) (laser fluence of 110 mJ/ cm²) times the sham control level at 2 weeks after laser photostimulation.

The expression level of EGF receptor was 1.11±0.02 (P=0.0013, n=3) (laser fluence of 55 mJ/ cm²) and 1.89±0.21 (P=0.004, n=3) (laser fluence of 110 mJ/ cm²) times the sham control level at 6 hours, then decreased to 1.11±0.005 (P=5.9E-06, n=3) (laser fluence of 55 mJ/ cm²) and 1.67±0.09 (P=0.00053, n=3) (laser fluence of 110 mJ/ cm²) times the sham control level at 24 hours, to 1.10±0.14 (P=0.358, n=3) (laser fluence of 55 mJ/ cm²) and 1.48±0.01 (P=6.46E-07, n=3) (laser fluence of 110mJ/cm²) the sham control level at 2 weeks after laser irradiation. (FIG 8B)

The gene expression of VEGF had also increased slightly by 6 hours, then gradually decreased to a level lower than that of sham control. Thus, the level was 1.58±0.12 (P=0.0024, n=3) (laser fluence of 55 mJ/ cm²) and 2.14±0.20 (P=0.00134, n=3) (laser fluence of 110 mJ/ cm²)times the sham control level at 6 hours, but then dropped to 0.93±0.17 (P=0.65, n=3) (laser fluence of 55 mJ/ cm²) and 0.89±0.09 (P=0.125, n=3) (laser fluence of 110 mJ/ cm²) times the sham control level times at 24 hours, to 0.90±0.02 (P=0.0027, n=3) (laser fluence of 55 mJ/ cm²) and 0.89±0.08 (P=4.47E-05, n=3) (laser fluence of 110 mJ/ cm²) times the sham control level at 2 weeks after selective photostimulation. (FIG 8B)

After selective photostimulation, an up-regulation of pigment epithelium-derived factor (PEDF) was observed in the pigmented ARPE-19 cells at 6 hours, after which the level of PEDF gradually declined. The level was 1.19±0.06 (P=0.008, n=3) (laser fluence of 55 mJ/ cm²) and 1.59±0.33 (P=0.07, n=3) (laser fluence of 110 mJ/ cm²) times the sham control level at 6 hours, then reduced to 1.07±0.03 (P=0.025, n=3) (laser fluence of 55 mJ/ cm²) and 1.36±0.07 (P=0.0032, n=3) (laser fluence of 110 mJ/ cm²)times the sham control level at 24 hours, then to 1.06±0.026 (P=0.027, n=3) (laser fluence of 55 mJ/ cm²) and 1.34±0.09 (P=0.005, n=3) (laser fluence of 110 mJ/ cm²)times the sham control level at two weeks after laser irradiation. (FIG 8B)

It should be noted that heat shock proteins Hsp27, Hsp70, and Hsp90 showed a transient increase of expression at 6 hours after selective photostimulation and then gradually decreased. Thus, the level of Hsp27 was 1.17±0.04 (P=0.00053, n=3) (laser fluence of 55 mJ/ cm²) and 1.59±0.12 (P=0.0003, n=3) (laser fluence of 110 mJ/ cm²) times the sham control level at 6 hours, but then dropped to 1.03±0.04 (P=0.364, n=3) (laser fluence of 55 mJ/ cm²) and 1.25±0.123 (P=0.047, n=3) (laser fluence of 110 mJ/ cm²) times the sham control level times at 24 hours, to 1.05±0.033 (P=0.124, n=3) (laser fluence of 55 mJ/ cm²) and 1.16±0.033 (P=0.00245, n=3) (laser fluence of 110 mJ/ cm²) times the sham control level at 2 weeks after selective photostimulation.

The level of Hsp70 was 1.24±0.08 (P=0.01, n=3) (laser fluence of 55 mJ/ cm²) and 1.42±0.03 (P=0.000053, n=3) (laser fluence of 110 mJ/ cm²) times the sham control level at 6 hours, but then dropped to 1.37±0.09 (P=0.006, n=3) (laser fluence of 55 mJ/ cm²) and 1.82±0.25 (P=0.01, n=3) (laser fluence of 110 mJ/ cm²) times the sham control level times at 24 hours, to 1.17±0.14 (P=0.124, n=3) (laser fluence of 55 mJ/ cm²) and 1.68±0.09 (P=0.0004, n=3) (laser fluence of 110 mJ/ cm²) times the sham control level at 2 weeks after selective photostimulation.

The level of Hsp90 was 1.44±0.24 (P=0.06, n=3) (laser fluence of 55 mJ/ cm²) and 1.97±0.21 (P=0.003, n=3) (laser fluence of 110 mJ/ cm²) times the sham control level at 6 hours, but then dropped to 1.08±0.04 (P=0.47, n=3) (laser fluence of 55 mJ/ cm²) and 1.22±0.08 (P=0.02, n=3) (laser fluence of 110 mJ/ cm²) times the sham control level times at 24 hours, to 1.05±0.04 (P=0.118, n=3) (laser fluence of 55 mJ/ cm²) and 1.18±0.06 (P=0.02, n=3) (laser fluence of 110 mJ/ cm²) times the sham control level at 2 weeks after selective photostimulation. (FIG 8B)

These results demonstrate that selective photostimulation increased the expressions of PDGF, TGFβ1, bFGF, EGF, IGF, and their receptors, VEGF, PEDF, and heat stock proteins after laser irradiation. Growth factors regulate many of the processes crucial for normal wound healing. For example, TGF-*β* is a chemoattractant for inflammatory cells and promotes matrix deposition. PDGF, EGF, and FGF promote RPE cell proliferation. IGF-1 is a potent cell survival factor as well as being strongly implicated in the pathobiology of preretinal neovascularization. It seems likely that these growth factors work in harmony to elicit wound repair. The presented experiments show a temporal variation in growth factor expression in the RPE following selective photostimulation which infers that RPE wound healing after selective photostimulation is regulated by growth factors in an autocrine/paracrine manner. Up-regulation of anti-angiogenic factors such as PEDF and Hsps after selective photostimulation should contribute to the beneficial effects of selective photostimulation.

### Example 7: Sublethal Pulsed Laser Treatment Induces Enhanced Regeneration in Retinal Pigment Epithelial Cells

This example provides an evaluation of the biological response of human fetal retinal pigmented epithelial (RPE) cells to pulsed laser treatment to develop a selective and safe laser treatment regimen for improved RPE regeneration.

Human fetal retinal pigmented epithelial (hfRPE) cells were isolated from 19-week old fetal eyes as described by Maminishkis et al., "Confluent monolayers of cultured human fetal retinal pigment epithelium exhibit morphology and physiology of native tissue," Invest. Ophthalmol. Vis. Sci., 47(8):3612-24 (2006), and cultured on laminin coated glass slides for 4 weeks until they become confluent and darkly pigmented. Human fetal RPE cells were treated with a pulsed dye laser (Palomar 3010; 590 nm, 1 µs, 1 mm dia). The cells were irradiated with a single pulse laser at fluences between 50-700 mJ/cm² and were analyzed using a fluorescent live/dead stain (Invitrogen, Carlsbad, CA) two hours after the treatment. The fluorescent images of stained cells were used to determine the number of live and dead cells within the irradiation field using the METAMORPH® software (Molecular Devices, Sunnyvale, CA).

For this example, the laser energy level that caused 50% cell death within the irradiation area was assigned to be the threshold energy level and sub-threshold energy was defined as the energy level where no cell death was observed. The experiments were performed at an energy level that is 70% of the sub-threshold energy. The regeneration response of hfRPE cells upon single pulse laser treatment was evaluated in an in vitro scratch wound healing model. The expression of mitogenic growth factors such as platelet derived growth factor (PDGF), fibroblast growth factor (bFGF), and insulin-like growth factor (IGF1 and IGF2) two hours post laser treatment was analyzed via reverse transcriptase polymerase chain reaction (RT-PCR).

Using the 1-µs single pulse dye laser, the threshold energy level was determined to be 496 mJ/cm² and the sub-threshold energy level was determined to be 285 mJ/cm² for hfRPE cells. The experiments were performed at 200 mJ/cm² (70% of sub-threshold energy). In the scratch type in vitro wound healing model, there was approximately 20-fold increase in proliferation of hfRPE cells following single pulse dye laser irradiation when compared to sham controls. In addition, following laser treatment growth factor expression was upregulated as follows: bFGF (1.96-fold), IGF1 (4.6-fold), IGF2 (2.2-fold) and PDGF (0.14-fold), as analyzed by RT-PCR and densitometry.

The results indicate that sub-lethal pulsed laser treatment of hfRPE cells stimulate expression of mitogenic factors that lead to enhanced proliferation in an in vitro wound healing model. Focal sub-lethal pulsed laser treatment of the RPE preserves surrounding cells and can be used to improve RPE regeneration in diseases implicated by RPE degeneration.

### Example 8 - Viability Study of RPE Cells using Different Lasers

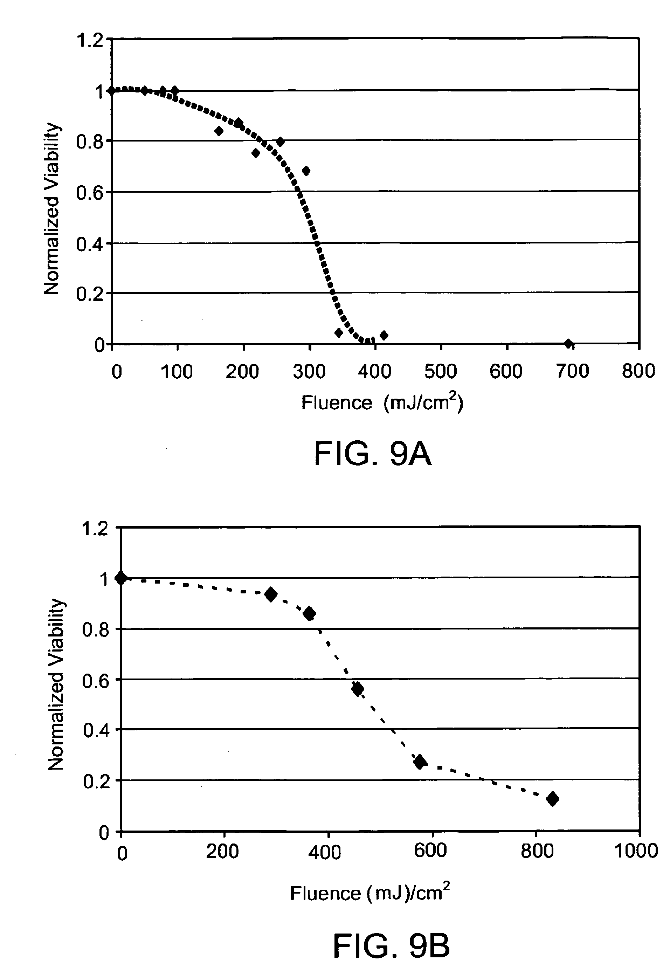

FIGS. 9A and 9B show the results of a viability study of human fetal RPE (hfRPE) cells after administering a single pulse with a Nd:YAG laser (pulse duration of about 3 ns) and a pulsed dye laser (pulse duration of about 1 µs), respectively. For the YAG laser, normalized viability values were at about 100% to 95% viability at a fluence up to about 100 mJ/cm², a viability of above 80% at a fluence of up to about 200 mJ/cm², and steep drop-off in viability to below 70% at a fluence of about 300 mJ/cm². For the pulse dye laser, a normalized viability of over 90% at a fluence of about 300 mJ/cm², a viability over 80% at a fluence of about 380-390 mJ/cm², and a viability of about 50% at a fluence of about 500 mJ/cm².

Thus, for ns-pulse durations, FIG 9A shows that exposing hfRPE cells with a fluence of up to several tens of mJ essentially causes no killing. Even a pulse fluence between 100 mJ/cm² and about 250 mJ/cm² causes only a small reduction in normalized viability of the hfRPE cells.

For µs-pulse durations, FIG. 9B shows that administering hfRPE cells with a fluence of up to several hundreds of mJ essentially causes essentially no cell death. Even a pulse fluence of about 250 mJ causes only small reduction in viability of the hfRPE cells.

These results show that administering fluences from nJ to several mJ can ensure high viability without significant cell death of hfRPE cells.

Even though melanin concentration was not controlled, a higher melanin concentration would lower the fluence threshold. Thus, using 70% of the upper limit of no cell death helps assure that there is no (or essentially no) killing of cells.

### Example 9 - In Vivo Rabbit Model of Sublethal Fluence Levels

Using a Mainster contact lens placed onto the cornea of a Dutch Belted Rabbits, RPE cells were irradiated with a fluence of about 64 mJ/cm² using a 1 µs pulsed dye laser and of about 8.0 mJ/cm² using a q-switched Nd:YAG laser at 532 nm. No visible damage to the RPE was observed. However, fluorescein angiography demonstrated leakage at the above fluences, indicating opening of junctions between cells, and thus providing evidence of a biochemical effect induced by the light energy without ophthalmoscopically visible changes in the RPE.

This data suggests that photostimulation of the RPE in vivo occurs at lower treatment energies than that reported using RPE cells in culture.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A system for selectively photostimulating retinal pigmented epithelial cells in a tissue in a patient, the system comprising:
a light source (7) for generating one or more radiation pulses, wherein the light source is a Nd-based laser and wherein each pulse comprises:
(i) a light having a wavelength of about 532 nm that is absorbed more in pigmented cells than in nonpigmented cells; and
(ii) a pulse duration of about 3ns that is shorter than a thermal relaxation time of the retinal pigmented epithelial cells;
an optical system for directing the one or more pulses to a region of tissue containing retinal pigmented epithelial cells and nonpigmented cells,
wherein the pigment is either endogenously synthesized or exogenous pigment; and
a control unit (8) configured to control the irradiating of the tissue with the one or more radiation pulses such that the total radiation energy applied to the tissue provides a sublethal fluence to the retinal pigmented epithelial cells of between 100 mJ/cm² and 250 mJ/cm², thereby selectively photostimulating the retinal pigmented epithelial cells in the tissue.

2. The system of claim 1, wherein the control unit (8) is configured to selectively photostimulate the retinal pigmented epithelial cells while avoiding cell death.

3. The system of claim 1, wherein the control unit (8) is further configured to control at least one of a pulse duration, a wavelength, and a total number of radiation pulses generated by the light source (7) to apply the sublethal energy fluence to the retinal pigmented epithelial cells.

4. The system of claim 1, wherein the control unit (8) is further configured to selectively photostimulate the retinal pigmented epithelial cells in the tissue by:
(i) activating the light source (7) to generate one or more radiation pulses for irradiating the tissue during a first treatment period;
(ii) de-activating the light source (7) for a healing period of one hour or more; and
(iii) re-activating the light source (7) to generate one or more additional radiation pulses to further irradiate the tissue during a second treatment period.

5. The system of claim 3, wherein the light source (7) is a frequency-doubled Nd:YAG laser, and wherein the control unit (8) is configured to induce proliferation of the retinal pigmented epithelial cells by:
adjusting the light source (7) to generate radiation pulses; and
applying the radiation pulses to the retinal pigmented epithelial cells,
wherein each of the radiation pulses is separated by a delay time to ensure that photocoagulation does not occur in the tissue.

6. The system of claim 5, wherein the control unit (8) is configured to apply a single radiation pulse to the retinal pigmented epithelial cells to induce proliferation of the cells.

7. The system of claim 4, wherein the control unit (8) is further configured to selectively photostimulate the retinal pigmented epithelial cells in the tissue by
(ii) deactivating the light source (7) for one or more additional healing periods; and
(iii) reactivating the light source (7) following each of the additional healing periods to generate one or more additional radiation pulses to further irradiate the tissue during one or more additional treatment periods.

8. The system of claim 4, wherein the control unit (8) is configured to activate the light source (7) to generate radiation pulse sequences of between 10 and 100 pulses during each of the first and second treatment periods.

9. The system of claim 8, wherein the control unit (8) is configured to activate the light source (7) to generate different numbers of radiation pulses during each of the first and second treatment periods.

10. The system of claim 1, wherein the one or more radiation pulses impinge upon the tissue in a target spot having a diameter of between about 0.05 mm and about 1.5 mm.

11. The system of claim 1, wherein the light source (7) is a Nd:YAG laser, and wherein the wavelength of the light corresponds to a frequency-doubling of a fundamental output wavelength of the Nd:YAG laser.

## Patentansprüche

1. System zur selektiven Fotostimulation retinaler Pigmentepithelzellen in einem Gewebe in einem Patienten, wobei das System umfasst:
eine Lichtquelle (7) zum Erzeugen eines oder mehrerer Strahlungspulse,
wobei die Lichtquelle ein Nd-basierter Laser ist und wobei jeder Puls umfasst:
(i) ein Licht mit einer Wellenlänge von ungefähr 532 nm, das stärker in pigmentierten Zellen absorbiert wird als in nichtpigmentierten Zellen; und
(ii) eine Pulsdauer von ungefähr 3 ns, die kürzer ist als die thermische Relaxationszeit der retinalen Pigmentepithelzellen;
ein optisches System, um den einen oder die mehreren Pulse auf eine Region des Gewebes, welche retinale Pigmentepithelzellen und nichtpigmentierte Zellen enthält zu richten, wobei das Pigment entweder endogen gebildet ist oder ein exogenes Pigment ist; und
eine Steuereinheit (8), ausgelegt, um die Bestrahlung des Gewebes mit dem einen oder den mehreren Strahlungspulsen zu steuern, so dass die gesamte Strahlungsenergie, die auf das Gewebe appliziert wird eine subletale Fluenz zwischen 100 mJ/cm² und 250 mJ/cm² an die retinalen Pigmentepithelzellen liefert, wodurch die Pigmentepithelzellen in dem Gewebe selektiv fotostimuliert werden.

2. System nach Anspruch 1, wobei die Steuereinheit (8) ausgelegt ist, um die retinalen Pigmentepithelzellen selektiv zu fotostimulieren wobei ein Absterben der Zellen vermieden wird.

3. System nach Anspruch 1, wobei die Steuereinheit (8) außerdem ausgelegt ist, um zumindest eines zu steuern aus einer Pulsdauer, einer Wellenlänge und einer Gesamtanzahl an Strahlungspulsen, die von der Lichtquelle (7) erzeugt werden, um die subletale Energiefluenz auf die retinalen Pigmentepithelzellen zu applizieren.

4. System nach Anspruch 1, wobei die Steuereinheit (8) außerdem ausgelegt ist, um die retinalen Pigmentepithelzellen selektiv zu fotostimulieren, durch:
(i) Aktivieren der Lichtquelle (7), um einen oder mehrere Strahlungspulse für die Bestrahlung des Gewebes während einer ersten Behandlungsperiode zu erzeugen;
(ii) Deaktivieren der Lichtquelle (7) für eine Heilperiode von einer Stunde oder mehr; und
(iii) Reaktivieren der Lichtquelle (7), um einen oder mehrere zusätzliche Strahlungspulse zu erzeugen, um das Gewebe während einer zweiten Behandlungsperiode weiter zu bestrahlen.

5. System nach Anspruch 3, wobei die Lichtquelle (7) ein frequenzverdoppelter Nd:YAG Laser ist, und wobei die Steuereinheit (8) ausgelegt ist, um Proliferation der retinalen Pigmentepithelzellen hervorzurufen, durch:
Einstellen der Lichtquelle (7), um Strahlungspulse zu erzeugen; und
Applizieren der Strahlungspulse auf die retinalen Pigmentepithelzellen,
wobei die Strahlungspulse jeweils durch eine Verzögerungszeit voneinander getrennt sind, um sicherzustellen, dass in dem Gewebe keine Fotokoagulation auftritt.

6. System nach Anspruch 5, wobei die Steuereinheit (8) ausgelegt ist, um einen einzelnen Strahlungspuls auf die retinalen Pigmentepithelzellen zu applizieren, um Proliferation der Zellen hervorzurufen.

7. System nach Anspruch 4, wobei die Steuereinheit (8) außerdem ausgelegt ist, um die retinalen Pigmentepithelzellen in dem Gewebe selektiv zu fotostimulieren, durch:
(ii) Deaktivieren der Lichtquelle (7) für eine oder mehrere zusätzliche Heilperioden; und
(iii) Reaktivieren der Lichtquelle (7) in Anschluss an jede der zusätzlichen Heilperioden, um einen oder mehrere zusätzliche Strahlungspulse zu erzeugen, um das Gewebe während einer oder mehrerer zusätzlicher Behandlungsperioden zu bestrahlen.

8. System nach Anspruch 4, wobei die Steuereinheit (8) ausgelegt ist, um die Lichtquelle (7) zu aktivieren, um Strahlungspuls-Sequenzen zwischen 10 und 100 Pulsen währen jeder der ersten und zweiten Behandlungsperioden zu erzeugen.

9. System nach Anspruch 8, wobei die Steuereinheit (8) ausgelegt ist, um die Lichtquelle (7) zu aktivieren, um unterschiedliche Anzahlen an Strahlungspulsen während jeder der ersten und zweiten Behandlungsperioden zu erzeugen.

10. System nach Anspruch 1, wobei die einen oder mehreren Strahlungspulse auf dem Gewebe in einem Zielbereich mit einem Durchmesser zwischen ungefähr 0,05 mm und ungefähr 1,5 mm auftreffen.

11. System nach Anspruch 1, wobei die Lichtquelle (7) ein Nd:YAG Laser ist, und wobei die Wellenlänge des Lichts einer Frequenzverdoppelung einer Fundamentalausgangswellenlänge des Nd:YΔG Lasers entspricht.

## Revendications

1. Système de photostimulation sélective de cellules épithéliales pigmentées rétiniennes dans un tissu chez un patient, le système comprenant :
une source de lumière (7) pour générer une ou plusieurs impulsions de rayonnement, la source de lumière étant un laser au néodyme, et chaque impulsion comprenant
(i) une lumière ayant une longueur d'onde d'environ 532 nm qui est plus absorbée dans des cellules pigmentées que dans des cellules non pigmentées ; et
(ii) une durée d'impulsion d'environ 3 ns qui est plus courte que le temps de relaxation thermique des cellules épithéliales pigmentées rétiniennes ;
un système optique pour diriger la ou les impulsions vers une région de tissu contenant des cellules épithéliales pigmentées rétiniennes et des cellules non pigmentées, le pigment étant synthétisé par voie endogène ou étant un pigment exogène ; et
une unité de commande (8) configurée pour contrôler l'irradiation du tissu avec la ou les impulsions de rayonnement de telle sorte que l'énergie de rayonnement totale appliquée au tissu fournit une fluence sublétale aux cellules épithéliales pigmentées rétiniennes comprise entre 100 mJ/cm² et 250 mJ/cm², pour photostimuler ainsi sélectivement les cellules épithéliales pigmentées rétiniennes dans le tissu.

2. Système selon la revendication 1, dans lequel l'unité de commande (8) est configurée pour photostimuler sélectivement les cellules épithéliales pigmentées rétiniennes tout en évitant la mort des cellules.

3. Système selon la revendication 1, dans lequel l'unité de commande (8) est en outre configurée pour contrôler au moins un paramètre parmi la durée des impulsions, la longueur d'onde et le nombre total d'impulsions de rayonnement générées par la source de lumière (7) afin d'appliquer la fluence énergétique sublétale aux cellules épithéliales pigmentées rétiniennes.

4. Système selon la revendication 1, dans lequel l'unité de commande (8) est en outre configurée pour photostimuler sélectivement les cellules épithéliales pigmentées rétiniennes dans le tissu :
(i) en activant la source de lumière (7) pour générer une ou plusieurs impulsions de rayonnement afin d'irradier le tissu pendant une première période de traitement ;
(ii) en désactivant la source de lumière (7) pendant une période de cicatrisation d'au moins une heure ; et
(iii) en réactivant la source de lumière (7) pour générer une ou plusieurs impulsions de rayonnement supplémentaires afin d'irradier encore le tissu pendant une deuxième période de traitement.

5. Système selon la revendication 3, dans lequel la source de lumière (7) est un laser Nd:YAG à fréquence doublée, et dans lequel l'unité de commande (8) est configurée pour induire une prolifération des cellules épithéliales pigmentées rétiniennes :
en réglant la source de lumière (7) pour générer des impulsions de rayonnement ; et
en appliquant les impulsions de rayonnement aux cellules épithéliales pigmentées rétiniennes, chacune des impulsions de rayonnement étant séparée par un temps d'attente afin de garantir qu'une photocoagulation ne se produit pas dans le tissu.

6. Système selon la revendication 5, dans lequel l'unité de commande (8) est configurée pour appliquer une seule impulsion de rayonnement aux cellules épithéliales pigmentées rétiniennes afin d'induire une prolifération des cellules.

7. Système selon la revendication 4, dans lequel l'unité de commande (8) est en outre configurée pour photostimuler sélectivement les cellules épithéliales pigmentées rétiniennes dans le tissu :
(ii) en désactivant la source de lumière (7) pendant une ou plusieurs périodes de cicatrisation supplémentaires ; et
(iii) en réactivant la source de lumière (7) après chacune des périodes de cicatrisation supplémentaires pour générer une ou plusieurs impulsions de rayonnement supplémentaires afin d'irradier encore le tissu pendant une ou plusieurs périodes de traitement supplémentaires.

8. Système selon la revendication 4, dans lequel l'unité de commande (8) est configurée pour activer la source de lumière (7) afin de générer des séquences d'impulsions de rayonnement de 10 à 100 impulsions pendant chacune des première et deuxième périodes de traitement.

9. Système selon la revendication 8, dans lequel l'unité de commande (8) est configurée pour activer la source de lumière (7) afin de générer des nombres différents d'impulsions de rayonnement pendant chacune des première et deuxième périodes de traitement.

10. Système selon la revendication 1, dans lequel la ou les impulsions de rayonnement frappent le tissu en un point cible ayant un diamètre compris entre environ 0,05 mm et environ 1,5 mm.

11. Système selon la revendication 1, dans lequel la source de lumière (7) est un laser Nd:YAG, et dans lequel la longueur d'onde de la lumière correspond à un doublement de fréquence d'une longueur d'onde de sortie fondamentale du laser Nd:YAG.
